# EUROPEAN PATENT APPLICATION

(11) **EP 1 801 224 A1**
(43) Date of publication of application: **27.06.2007**
(21) Application number: 05768431.8
(22) Date of filing: 04.08.2005
(51) Int. Cl.: C12P 7/42, C12P 7/62, C07B 53/00, C07C 51/367, C07C 51/09, C07C 59/64, C07C 67/11, C07C 69/734, C12N 9/04

(54) **PROCESS FOR PRODUCING OPTICALLY ACTIVE 2-SUBSTITUENT-OXY-3-(4-SUBSTITUENT-OXYPHENYL)PROPIONIC ACID DERIVATIVE**

(30) Priority: 13.08.2004 JP 2004236154
(71) Applicant: Kaneka Corporation, Kita-ku Osaka-shi, Osaka 530-8288 (JP)
(72) Inventor: HONDA, Tatsuya,c/o KANEKA CORPORATION, Takasago-shi, Hyogo 6768688 (JP); MAEDA, Hirofumi, c/o KANEKA CORPORATION, Takasago-shi, Hyogo 6768688 (JP); NAGASHIMA, Nobuo, c/o KANEKA CORPORATION, Takasago-shi, Hyogo 6768688 (JP); KAWANO, Shigeru, c/o KANEKA CORPORATION, Takasago-shi, Hyogo 6768688 (JP); YASOHARA, Yoshihiko, c/o KANEKA CORPORATION, Takasago-shi, Hyogo 6768688 (JP)
(74) Representative: Vossius & Partner
(86) International application number: PCT/JP2005/014290
(87) International publication number: WO 2006/016517

(57) **Abstract**

The present invention relates to a process for producing an optically active
2-substituent-oxy-3-(4-substituent-oxyphenyl)propionic acid derivative which comprises stereoselectively reducing an 2-oxo-3-(4-substituent-oxyphenyl)propionic acid by an enzyme and subjecting the thus-obtained optically active 2-hydroxy-3-(4'-substituent-oxyphenyl)propionic acid to esterification of the carboxyl group according to need, then to alkylation of the hydroxyl group and, if necessary, to deprotection of an ether type protective group. The present invention may make it possible to produce an optically active 2-subsituent-oxy-3-(4-substituent-oxyphenyl)propionic acid derivative, which is useful as intermediates for the synthesis of medicinal compounds, efficiently, in a simple and easy manner, and commercially advantageously.

## Description

### TECHNICAL FIELD

The present invention relates to a process for producing optically active 2-subsituent-oxy-3-(4-substituent-oxyphenyl)propionic acid derivatives, which are very useful as intermediates for the synthesis of medicinal compounds, agrochemical compounds and the like, and important intermediates therefor and to those important intermediates.

### BACKGROUND ART

Known as the prior art processes for producing optically active 2-substituent-oxy-3-(4-substituent-oxyplienyl)p.ropionic acid derivatives are such processes as shown below.
(1) The process comprising carrying out asymmetric hydrolysis by causing an enzyme to act on racemic ethyl -2-ethoxy-3-(4-hydroxyphenyl)propionate and separating the optically active form (Patent Document 1).
(2) The process comprising causing optically active phenethylamine to act on racemic 2-hydroxy-3-(4-benzyloxyphenyl)propionic acid to give diastereomer salts, separating one optically active form by recrystallization and synthesizing optically active ethyl 2-ethoxy-3-(4-hydroxyphenyl)propionate via alkylation of the hydroxyl group and carboxyl group (Patent Document 2).
(3) The process comprising using L-tyrosine as a starting material and synthesizing optically active ethyl 2-ethoxy-3-(4-hydroxyphenyl)propionate via the nitrous acid-induced degradation reaction (Patent Document 2).

However, the process (1) is not economically advantageous since the number of steps for synthesizing racemic ethyl 2-ethoxy-3-(4-hydroxyphenyl)propionate is large and the final step is an optical resolution step and therefore the yield is low and the productivity is also low. Like the process (1), the process (2) is not economically advantageous since it includes a large number of steps for synthesizing the racemic form and involves an optical resolution step, so that the yield is low and the productivity is also low. The process (3) is not advantageous from the industrial viewpoint since it uses isoamyl nitrite, which needs caution in handling. Further, in the nitrous acid-induced degradation reaction, the optical purity may possibly be lowered and, therefore, some or other means for improving the optical purity is required and, in addition, the process is low in yield. Furthermore the number of all steps is large and thus the process is not economically advantageous.

Thus, when considered as commercial processes, the prior art processes all have problems to be solved. Therefore, in view of the above-discussed state of the art, it is an object of the present invention to provide a process for producing optically active 2-substituent-oxy-3-(4-substituent-oxyphenyl)propionic acid derivatives which is efficient and economical and can be carried out suitably on a commercial scale, as well as important intermediates therefor.
Patent Document 1: WO 01/11073
Patent Document 2: WO 00/26200

### SUMMARY OF THE INVENTION

The present inventors made various investigations to accomplish such object as mentioned above and, as a result, they have now completed the present invention.

Thus, the invention provides
a process for producing an optically active 2-substituent-oxy-3-(4-substituent-oxyphenyl)propionic acid derivative represented by the general formula (3): 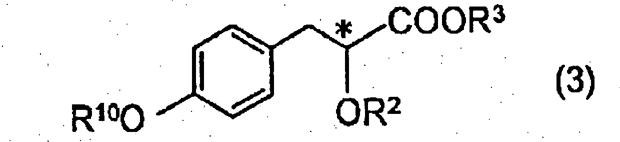 (wherein * indicates an asymmetric carbon atom, R¹⁰ represents a hydrogen atom or an ether type protective group represented by R¹, R² represents an alkyl group containing 1 to 10 carbon atoms, which may optionally be substituted, or an aralkyl group containing 7 to 20 carbon atoms, which may optionally be substituted, R³ represents a hydrogen atom, an alkyl group containing 1 to 10 carbon atoms, which may optionally be substituted, an aryl group containing 6 to 20 carbon atoms, which may optionally be substituted, or an aralkyl group containing 7 to 20 carbon atoms, which may optionally be substituted)
which comprises stereoselectively reducing an 2-oxo-3-(4-substituent-oxyphenyl)propionic acid represented by the general formula (1): 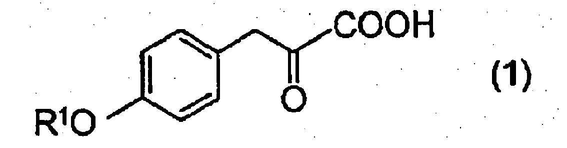 (wherein R¹ represents an ether type protective group) by means of an enzyme, and subjecting the thus-obtained optically active 2-hydroxy-3-(4-substituent-oxyphenyl)propionic acid represented by the general formula (2): 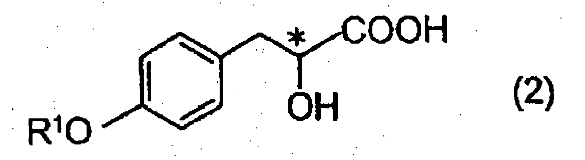 (wherein * and R¹ are as defined above) to esterification of the carboxyl group according to need, then to alkylation of the hydroxyl group and, if necessary, to elimination of R¹ (deprotection).

The invention also provides
a process for selectively producing an optically active 2-substituent-oxy-3-(4-substituent-oxyphenyl)propionic acid represented by the general formula (4): 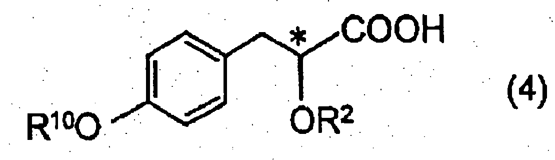 (wherein *, R¹⁰ and R² are as defined above)
which comprises selectively alkylating the hydroxyl group of an optically active 2-hydroxy-3-(4-substituent-oxyphenyl)propionic acid represented by the general formula (2) given above with an alkyl halide in an ether type solvent and/or a nitrile type solvent in the presence of a base, if necessary followed by elimination of R¹ (deprotection).

Further, the invention provides
2-oxo-3-(4-tert-butoxyphenyl)propionic acid represented by the general formula (7): 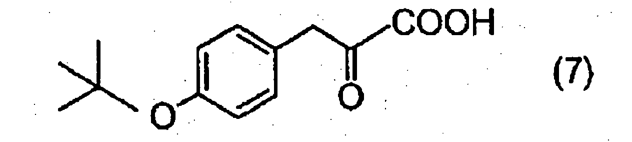 and an optically active
2-substituent-oxy-3-(4-tert-butoxyphenyl)propionic acid derivative represented by the general formula (8) 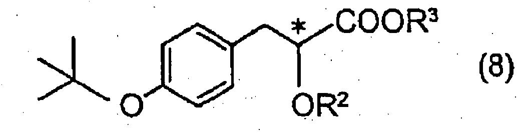 (wherein *, R² and R³ are as defined above).

### (Effect of the invention)

According to the present invention, which has the above-mentioned constitutions, optically active 2-substituent-oxy-3-(4-substituent-oxyphenyl)propionic acid derivatives or optically active
2-substituent-oxy-3-(4-hydroxyphenyl)propionic acid derivatives can be produced from
2-oxo-3-(4-substituent-oxyphenyl)propionic acids efficiently, in a simple and easy manner, and commercially advantageously.

In the following, the invention is described in detail.

### DETAILED DESCRIPTION OF THE INVENTION

First, the step of producing an optically active 2-hydroxy-3-(4-substituent-oxyphenyl)propionic acid (2) represented by the general formula (2): 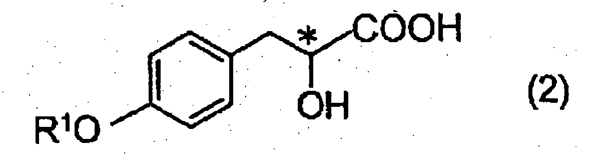 by stereoselectively reducing the corresponding 2-oxo-3-(4-substituent-oxyphenyl)propionic acid represented by the general formula (1): 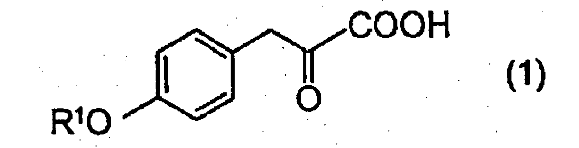 using an enzyme is described.

In the above formulae (1) and (2), R¹ represents an ether type protective group, including, for example, methyl, tert-butyl, benzyl, allyl, methoxymethyl, methoxyethoxymethyl, tetrahydropyranyl, benzyloxymethyl, methylthiomethyl, 2-(trimethylsilyl)ethoxymethyl, and tert-butoxymethyl groups, etc. Among them, methyl, tert-butyl, benzyl, allyl and methoxymethyl groups are preferred, and tert-butyl group is more preferred, since protection and deprotection can be realized in an easy and inexpensive manner. In the above formula (2), * indicates that the carbon atom marked therewith is an asymmetric carbon atom.

The compound represented by the formula (1) can be synthesized, for example, by subjecting the corresponding 4-substituent-oxybenzaldehyde to condensation with hydantoin to give the corresponding benzylidenehydantoin, followed by hydrolysis of the same.

The compound of the above formula (1) in which R¹ is a tert-butyl group, namely 2-oxo-3-(4-tert-butoxyphenyl)propionic acid represented by the general formula (7): 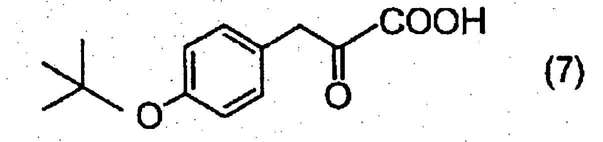 or salts thereof are novel compounds useful as intermediates for the synthesis of medicinal compounds.

The enzyme to be used in carrying out the above reaction may be any of those enzymes capable of asymmetrically reducing the compound represented by the formula (1) among the enzymes effective in reducing α-keto acids to optically active α-hydroxy acids, namely α-keto acid reducing enzymes. Such enzymes can all be used in the above reaction.

The α-keto acid reducing enzyme to be used in the practice of the invention may be of any origin; thus, it may be not only a microorganism-derived one but also an animal- or plant-derived one. The α-keto acid reducing enzyme to be used in the reaction may be a purified one having a high purity or microbial cells containing that enzyme or a product obtained by some or other treatment of microbial cells, for instance. Furthermore, a transformant derived from *Escherichia coli*, for instance, by introduction of a gene coding for the α-keto acid reducing enzyme in question in which transformant the enzyme in question has been expressed at a high level, or a product obtained by some or other treatment of such transformant may also be used. Thus, the enzyme in question can be used in the reaction without depending on the purity thereof.

Some of α-keto acid reducing enzymes are available on the market and can be obtained with ease. It is also possible to obtain some known α-keto acid reducing enzyme and use the same in the reaction or newly search one and use the enzyme thus found.

As the α-keto acid reducing enzyme, there may be mentioned, for example, L- or D-lactic acid dehydrogenase, L- or D-hydroxyisocaproic acid dehydrogenase, and L- or D-mandelic acid dehydrogenase, among others. Among these, either one of L- or D-lactic acid dehydrogenase is preferably used in the reaction. These may be known enzymes or enzymes newly found out.

Known as the L-lactic acid dehydrogenase are, for example, enzymes derived from the following microorganisms: *LactobacillusplantarumWSOstrain* (Food Microbiology, 3, 93-99 (1986)), *Lactobacillus curvatus* DSM20010 strain (Arch. Microbiol., 112, 81-93 (1977)), *Lactococcus lactis* subsp. *lactis* LM0230 strain (J. Bacteriol., 174, 6956-6964 (1992)), Streptococcus *bovis* JB1 strain (Current Microbiology, 34, 367-373 (1997)), *Bacillus stearothermophilus* NCA1503 (ATCC29609) strain (Gene, 46, 47-55 (1986)), *Bifidobacterium longum* aM101-2 (Gene, 85, 161-168 (1989) ) and. *Thermus thermophilus* HB8 strain (Appl. Microbiol. Biotechnol., 40, 676-681 (1994), among others. Also usable are bovine-, swine-, chicken-, rabbit- or human-derived ones, among others; these can be obtained with ease.

Known as the D-lactic acid dehydrogenase are, for example, enzymes derived from the following microorganisms: *Lactobacillus pentosus* ATCC8041 strain (J. Biol. Chem., 268, 12588-12594 (1991)), *Lactobacillus helveticus* ATCC15009 strain (Eur. J.Biochem., 208, 799-805 (1992)), *Lactobacillus leichmannii* NCDO302 strain (J. Gen. Microbiol., 62, 241-250 (1970), *Lactobacillus* confusus DSM20196 strain (Eur. J. Appl. Microbiol. Biotechnol., 18, 75-85 (1983)), *Leuconostoc. mesenteroides* subsp. *cremoris* 195 strain (Res. Microbiol., 146, 291-302 (1995)) , *Leuconostoc lactis* NCDO546 strain (Biochemie, 55, 104771056 (1973)) and *Staphylococcus epidermidis* (the enzyme derived therefrom is available from Sigma), among others.

As the L-hydroxyisocaproic acid dehydrogenase, there may be mentioned, for example; *Lactobacillus* confusus DSM20196 strain-derived L-hydroxyisocaproic acid dehydrogenase (Appl. Microbiol. Biotechnol., 19, 167-176 (1984)).

Known as the D-hydroxyisocaproic acid dehydrogenase are ones derived from *Lactobacillus casei* ssp. *pseudoplantarum* DSM20008 strain (Appl. Microbiol. Biotechnol., 21, 7-15 (1985)) and *Lactobacillus delbrueckii* subsp. *bulgaricus* NCIB11778 strain (Eur. J. Biochem., 224, 439-446 (1994)).

As the L-mandelic acid dehydrogenase, there may be mentioned, for example, *Rhodotorula graminis* ATCC20804 strain-derived one.

Known as the D-mandelic acid dehydrogenase are, for example, enzymes derived from the following microorganisms: *Enterococcus faecalis* IAM10071.strain (Appl. Environ. Microbiol., 68, 947-951 (2002)), *Streptococcus faecalis* IFO1296 strain (Agric. Biol. Chem., 50, 2621-2631 (1986)), *Lactobacillus curvatus* DSM20019 strain (Appl. Microbiol. Biotechnol., 28, 433-439 (1988)) and *Rhodotorula graminis* KGX39, strain (Biochem. J., 281, 211-218 (1992)).

In cases where a compound represented by the formula (1) is to be stereoselectively reduced using an α-keto acid reducing enzyme, NADH or NADPH is required as a coenzyme. While the reaction can be carried out by adding a necessary amount of NADH or NADPH to the reaction system, it is possible to markedly reduce the amount of the expensive coenzyme to be used by carrying out the reaction using an enzyme having the ability to convert the oxidized coenzyme (NAD⁺ or NADP⁺) to the reduced form (NADH or NADPH) (hereinafter referred to as "coenzyme regenerating ability") together with the substrate or, in other words, by using a coenzyme regenerating system in combination with the α-keto acid reducing enzyme.

Usable as the enzyme having coenzyme regenerating ability are hydrogenases, formic acid dehydrogenase alcohol dehydrogenase, glucose-6-phosphate dehydrogenase and glucose dehydrogenase. Preferably used are glucose dehydrogenase and formic acid dehydrogenase; glucose dehydrogenase is particularly preferred. Such a reaction can be carried out by adding a coenzyme regenerating system to the asymmetric reduction reaction system. Further, when a transformant as transformed with both a DNA coding for an α-keto acid reducing enzyme and a DNA coding for glucose dehydrogenase is used as a catalyst, the reaction can be carried out efficiently without separately preparing an enzyme having coenzyme regenerating ability and adding the same to the reaction system.

The asymmetric reduction of a compound represented by the formula (1) given above using an α-keto acid reducing enzyme can be carried out in the following manner, without any particular restriction thereto.

The compound of formula (1) is added to an appropriate buffer solution, for example 100 mM phosphate buffer, the α-keto acid reducing enzyme is added thereto together with NADH or NADPH in an amount at least equimolar to the substrate, and the reaction is allowed to proceed with stirring. Alternatively, the compound of formula (1), namely the substrate, is added to an appropriate buffer solution, an α-keto acid reducing enzyme, such a coenzyme as NAD⁺ or NADP⁺ and such a coenzyme regenerating system as mentioned above (e.g. glucose and glucose dehydrogenase, formic acid dehydrogenase and formic acid) are further added, and the reaction is allowed to proceed with stirring under pH adjustment.

These reactions are carried out at a temperature of 5 to 80°C, preferably 10 to 60°C, more preferably 20 to 40°C. During the reaction, the pH of the reaction mixture is maintained at 3 to 10, preferably 4 to 9,_more preferably 5 to 8. The reaction can be carried out either batchwise or continuously. In the case of batchwise operation, the reaction substrate can be added at a charge concentration of 0.01 to 60 % (w/v) preferably 0.1 to 40%, more preferably 0.5 to 20%. During the reaction, the substrate, namely the compound represented by the formula (1), may be supplementarily added from time to time.

The method of recovering the optically active compound (2) formed is not particularly restricted but when the reaction mixture with or without a separation process of microbial cells etc. is extracted with such a solvent as ethyl acetate, toluene, tert-butyl methyl ether, hexane or methylene chloride and the extract is purified by distillation and/or recrystallization or silica gel column chromatography, for instance, the optically active compound (2) can be easily obtained in a highly pure form.

The absolute configuration of the compound (2) obtained by this reaction may be either S or R but compounds whose configuration is S are more preferred.

Now, the step of producing an optically active 2-substituent-oxy-3-(4-substituent-oxyphenyl)propionic acid derivative represented by the general formula (3): 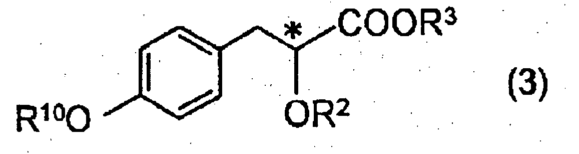 by esterifying the carboxyl group of the corresponding compound represented by the above formula (2) according to need and, then, alkylating the hydroxyl group, if necessary followed by elimination of R¹ (deprotection).

First, the substituents in the compound to be synthesized in this step are described.

In the compound (3) to be synthesized in this step, R² represents an alkyl group containing 1 to 10 carbon atoms, which may optionally be substituted, or an aralkyl group containing 7 to 20 carbon atoms, which may optionally be substituted, and thus is, for example, methyl, ethyl, n-propyl, isopropyl, n-butyl, isobutyl, tert-butyl, cyclohexyl, trifluoromethyl or benzyl group; among them, methyl and ethyl groups are preferred.

R³ represents a hydrogen atom, an alkyl group containing 1 to 10 carbon atoms; which may optionally be substituted, an aryl group containing 6 to 20 carbon atoms, which may optionally be substituted, or an aralkyl group containing 7 to 20 carbon atoms, which may optionally be substituted, and thus is, for example, a hydrogen atom, methyl, ethyl, n-propyl, isopropyl, n-butyl, isobutyl, tert-butyl, cyclohexyl, trifluoromethyl, phenyl or benzyl group; among them, a:hydrogen atom, methyl, ethyl groups and isopropyl groups are preferred, and a hydrogen atom, methyl and ethyl are more preferred.

R¹⁰ is the same as the above-mentioned R¹ or represents a hydrogen atom. Among them, tert-butyl group and a hydrogen atom are preferred, and a hydrogen atom is particularly preferred. The symbol * is as mentioned hereinabove.

Now, the reaction step in question is described.

In this reaction step, the hydroxyl group of the compound (2) is alkylated. Prior to the alkylation, the carboxyl group of the compound (2) may be esterified according to need. In this case, the phrase "according to need" means that the esterification of the carboxyl group of the compound (2) may be carried out or may not be carried out according to the reaction conditions and the compound desired. When the esterification is carried out, the methods of carboxyl group esterification as described in Protective Groups in Organic Synthesis, 3rd ed., Theodora W. Greene and Peter G. M. Wuts, Ed., Wiley-Interscience: New York, 1999; 373-428, for instance, can be used. As typical examples, there may be mentioned, for example, the method comprising subjecting the carboxylic acid to condensation with an alcohol in the presence of an acid catalyst, the method comprising reacting the carboxylic acid with an alcohol in the presence of a condensing agent, the method comprising reacting the carboxylic acid with an alkyl halide in the presence of a base, and the method comprising activating the carboxylic acid as the acid chloride or a mixed acid anhydride and reacting the activated form with an alcohol.

In particular when the ether type protective group R¹ in the compound (2) may possibly be decomposed under acidic conditions, the method using an alkyl halide is preferred since the esterification can then be carried out under neutral or alkaline conditions.

The reaction solvent to be used in this esterification reaction is not particularly restricted and may be any solvent incapable of adversely affecting the reaction. Thus, for example, mention may be made of hydrocarbon type solvents such as pentane, hexane, heptane, cyclohexane and petroleum ether, ester type solvents such as ethyl acetate and methyl acetate, aromatic hydrocarbon type solvents such as toluene, benzene and xylene, nitrile type solvents such as acetonitrile and propionitrile, ether type solvents such as tert-butyl methyl ether, diethyl ether, diisopropyl ether, tetrahydrofuran and dioxane, amide type solvents such as N,N-dimethylformamide and N,N-dimethylacetamide, sulfoxide type solvents such as dimethyl sulfoxide, halogenated hydrocarbon type solvents such as methylene chloride, 1,2-dichloroethylene, chloroform and carbon tetrachloride, carboxylic acid type solvents such as acetic acid and formic acid, alcohol type solvents such as methanol, ethanol and isopropanol, and water. These solvents may also be used in the form of mixtures of two or more of them. When a mixed solvent is used, the mixing ratio is not particularly restricted.

Although the concentration of the compound (2) in carrying out the reaction may vary depending on the reaction solvent used, the reaction can be carried out generally at a concentration of 1 to 50% by weight, preferably 5 to 30% by weight.

The reaction temperature in carrying out the reaction may vary depending on the acid or base etc. species used and the amount thereof and on the reaction solvent species used. Generally, however, it is within the range of from the freezing point to the boiling point of the reaction solvent. For driving the reaction to completion in a short period of time, it is recommended that the reaction be carried out at an elevated temperature and, from the side reaction inhibition viewpoint, it is recommended that the reaction temperature be set at a low level. Generally, the reaction temperature is preferably within the range of 40 to 100°C, more preferably -20 to 80°C.

The reaction time for the reaction may vary depending on the acid or base etc. species used and the amount thereof, the reaction solvent species and the reaction temperature. When the reaction is carried out at a temperature of -20 to 80°C, the reaction time is generally about 1 to 24 hours.

As for the after-treatment following this reaction, an ordinary treatment process for recovering the product from the reaction mixture can be employed. For example, water, hydrochloric acid or an aqueous alkali solution, for instance, is added to the reaction mixture after completion of the reaction for neutralization, followed by an extraction procedure using an ordinary solvent such as ethyl acetate, diethyl ether, methylene chloride, toluene or hexane. Distilling off the reaction solvent and extraction solvent from the extract obtained under reduced pressure gives the desired product. If necessary, the thus-obtained product may be subjected to an ordinary purification process such as silica gel chromatography, distillation and/or recrystallization to further increase the purity.

The alkylation reaction of the hydroxyl group of the compound (2) is now described. The alkylation of the hydroxyl group of the compound (2) can be carried out by causing an alkylating agent to act on the compound (2) in the presence of a base.

As the base to be used, there may be mentioned inorganic bases such as sodium hydroxide, sodium carbonate and potassium carbonate, alkali metal alkoxides such as sodium methoxide, sodium ethoxide, sodium isopropoxide and potassium tert-butoxide, alkali metal hydrides such as sodium hydride and potassium hydride, alkali metals such as metallic sodium, Grignard reagents such as tert-butylmagnesium chloride, n-butylmagnesium chloride and ethylmagnesium chloride, alkali metal amides such as lithium diisopropylamide and sodium amide, and organolithium compounds such as n-butyllithium, sec-butyllithium and tert-butyllithium. From the yield and economy viewpoint, sodium carbonate, sodium methoxide, sodium ethoxide, potassium tert-butoxide and sodium hydride are preferred among others.

The amount of the base to be used is not particularly restricted but, generally, the reaction can be carried out using the base in an amount of not smaller than 2 mole equivalents relative to the compound (2) or not smaller than 1 mole equivalent relative to the compound (2) in a salt form. Generally speaking from the economical viewpoint, the base is preferably used in an amount of not larger than 10.0 mole equivalents, more preferably not larger than 5.0 mole equivalents, still more preferably not larger than 4.0 mole equivalents.

The alkylating agent to be used includes alkyl halides such as methyl iodide, ethyl iodide, methyl bromide, ethyl bromide, benzyl bromide, methyl chloride, ethyl chloride and benzyl chloride, sulfuric esters such as dimethyl sulfate and diethyl sulfate, and sulfonic esters such as methyl methanesulfonate, ethyl methanesulfonate, methyl p-toluenesulfonate, ethyl p-toluenesulfonate and methyl p-trifluoromethanesulfonate. From the yield and economy viewpoint, methyl iodide, ethyl iodide, methyl bromide, ethyl bromide, benzyl bromide, benzyl chloride, methyl methanesulfonate, ethyl methanesulfonate, methyl p-toluenesulfonate, dimethyl sulfate and diethyl sulfate are preferred, and methyl iodide, ethyl iodide; dimethyl sulfate and diethyl sulfate are particularly preferred.

The amount of the alkylating agent to be used is not particularly restricted but, generally, the reaction can be carried out using an amount thereof of not smaller than 1 mole equivalent relative to the compound (2). Generally speaking from the economical viewpoint, the alkylating agent is used preferably in an amount of not larger than 10.0 mole equivalents, more preferably not larger than 5.0 mole equivalents, still more preferably not larger than 4.0 mole equivalents.

Generally, a solvent is used in the above reaction. The reaction solvent is not particularly restricted but may be any solvent incapable of adversely affecting the reaction. Thus, mention may be made of hydrocarbon type solvents such as pentane, hexane, heptane, cyclohexane and petroleum ether, ester type solvents such as ethyl acetate, methyl acetate, propyl acetate and methyl propionate, aromatic hydrocarbon type solvents such as toluene, benzene and xylene, nitrile type solvents such as acetonitrile and propionitrile, ether type solvents such as tert-butyl methyl ether, diethyl ether, diisopropyl ether, tetrahydrofuran and 1,4-dioxane, amide type solvents such as N,N-dimethylformamide and N,N-dimethylacetamide, sulfoxide type solvents such as dimethyl sulfoxide, halogenated hydrocarbon type solvents such as methylene chloride, 1,2-dichloroethylene, chloroform and carbon tetrachloride, and amine type solvents such as pyridine and triethylamine. These solvents may also be used in the form of mixtures of two or more.

From the reactant substrate solubility and yield viewpoint, toluene, acetonitrile, tetrahydrofuran, 1,4-dioxane, N,N-dimethylformamide and N,N-dimethylacetamide, and mixtures of two or more of these are preferred, and tetrahydrofuran and N,N-dimethylformamide are particularly preferred. In the case of use of a mixed solvent, the mixing ratio is not particularly restricted.

Although the concentration of the compound (2) in carrying out the reaction may vary depending on the reaction solvent used, the reaction can be carried out generally at a concentration of 1 to 50% by weight, preferably 5 to 30% by weight.

The reaction temperature in carrying out the reaction may vary depending on the base, alkylating agent and reaction solvent species employed. Generally, however, it is within the range of from the freezing point to the boiling point of the reaction solvent. For driving the reaction to completion in a short period of time, it is recommended that the reaction be carried out at an elevated temperature and, from the side reaction inhibition viewpoint, it is recommended that the reaction temperature be set at a low level. Generally, the reaction temperature is preferably within the range of -40 to 100°C, more preferably -20 to 80°C.

The reaction time for the reaction may vary depending on the base, alkylating agent and reaction solvent species used and on the reaction temperature. When the reaction is carried out at a temperature of -20 to 80°C, the reaction time is generally about 1 to 40 hours.

As for the after-treatment following this reaction, an ordinary treatment process for recovering the product from the reaction mixture can be employed. For example, water or hydrochloric acid, for instance, is added to the reaction mixture after completion of the reaction for neutralization, followed by an extraction procedure using an ordinary solvent such as ethyl acetate, diethyl ether, methylene chloride, toluene or hexane. Distilling off the reaction solvent and extraction solvent from the extract obtained under reduced pressure gives the desired product. If necessary, the thus-obtained product may be subjected to an ordinary purification process such as silica gel chromatography, distillation and/or recrystallization to further increase the purity.

In this step, after alkylation of the hydroxyl group, the protective group R¹ may be eliminated (for deprotection) according to need.

Here, the phrase "according to need" means that the protective group R¹ may be eliminated (for deprotection) or may not be eliminated according to the reaction conditions and the compound desired.

The deprotection conditions may vary according to the protective group used. When the protective group is methoxymethyl, methoxyethoxymethyl, tetrahydropyranyl, benzyloxymethyl, tert-butyl or methyl group, the deprotection reaction can be effected using an acid.

The acid to be used is not particularly restricted but includes mineral acids such as hydrochloric acid, hydrobromic acid and sulfuric acid, and organic acids such as trifluoroacetic acid, acetic acid, methanesulfonic acid and p-toluenesulfonic acid. Among them, hydrochloric acid is preferred from the economical viewpoint.

When the protective group is methyl group, the deprotection reaction can be carried out using a thiol such as methanethiol, ethanethiol or octanethiol.

The amount of the thiol to be used is not particularly restricted but, generally, the thiol is used in an amount of not smaller than 1 mole equivalent relative to the compound (3). Generally speaking from the economical viewpoint, that amount is preferably not larger than 10 mole equivalents, more preferably not larger than 3 mole equivalents.

The reaction solvent for the deprotection reaction is not particularly restricted and may be any solvent incapable of adversely affecting the reaction. Thus, for example, mention may be made of hydrocarbon type solvents such as pentane, hexane, heptane, cyclohexane and petroleum ether, ester type solvents such as ethyl acetate and methyl acetate, aromatic hydrocarbon type solvents such as toluene, benzene and xylene, nitrile type solvents such as acetonitrile and propionitrile, ether type solvents such as tert-butyl methyl ether, diethyl ether, diisopropyl ether, tetrahydrofuran and dioxane, amide type solvents such as N, N-dimethylformamide, N,N-dimethylace.tamide and N-methylpyrrolidinone; sulfoxide type solvents such as dimethyl sulfoxide, halogenated hydrocarbon type solvents such as methylene chloride, 1,2-dichloroethylene, chloroform and carbon tetrachloride, carboxylic acid type solvents such as acetic acid and formic acid, alcohol type solvents such as methanol, ethanol and isopropanol, and water. These solvents may also be used in the form of mixtures of two or more of them. When a mixed solvent is used, the mixing ratio is not particularly restricted.

Although the concentration of the compound (3) in carrying out the deprotection reaction may vary depending on the reaction solvent used, the reaction can be carried out generally at a concentration of 1 to 50% by weight, preferably 5 to 30% by weight.

The reaction temperature in the deprotection reaction may vary depending on the acid or thiol species used and the amount thereof and on the reaction solvent species used. Generally, it is within the freezing point to the boiling point of the reaction solvent used. For driving the reaction to completion in a short time, it is recommended that the reaction be carried out at an elevated temperature and, from the side reaction inhibition viewpoint, it is recommended that the reaction temperature be set at a low level. Generally, the reaction temperature is preferably within the range of 0 to 150°C, more preferably 20 to 120°C.

The reaction time for the deprotection reaction may vary depending on the acid or thiol species used and the amount thereof, the reaction solvent species used and the reaction temperature. When the reaction is carried out,at a temperature of 20 to 120°C, the reaction time is generally about 1 to 24 hours.

After the deprotection reaction, an ordinary treatment process for recovering the product from the reaction mixture can be employed. For example, water or an aqueous alkali solution, for instance, is added to the reaction mixture after completion of the reaction for neutralization, followed by an extraction procedure using an ordinary solvent such as ethyl acetate, diethyl ether, methylene chloride, toluene or hexane. Distilling off the reaction solvent and extraction solvent from the extract obtained under reduced pressure gives the desired product. If necessary, the thus-obtained product may be subjected to an ordinary purification process such as silica gel chromatography, distillation and/or recrystallization to further increase the purity.

When, in the reaction step from (2) to (3), the compound (2) is first esterified and then alkylated, a compound represented by the general formula (6): 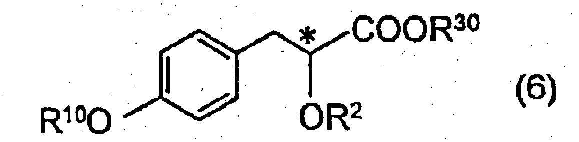 can be obtained as the compound (3) . In the compound (6), R², R¹⁰ and * are as defined above. R³⁰ represents an alkyl group containing 1 to 10 carbon atoms, which may optionally be substituted, an aryl group containing 6 to 20 carbon atoms, which may optionally be substituted, or an aralkyl group containing 7 to 20 carbon atoms, which may optionally be substituted, including methyl, ethyl, n-propyl, isopropyl, n-butyl, isobutyl, tert-butyl, cyclohexyl, trifluoromethyl, phenyl and benzyl groups, and others. Among them, methyl, ethyl and isopropyl groups are preferred, and isopropyl group is particularly preferred.

When the compound (2) is subjected to the above-mentioned alkylation reaction without preliminary esterification of the carboxyl group thereof, a compound represented by the general formula (4): 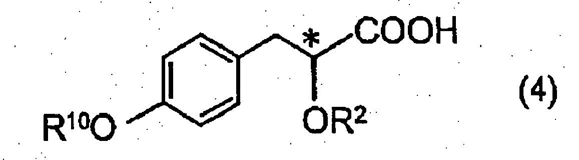 and/or a compound represented by the general formula (5): 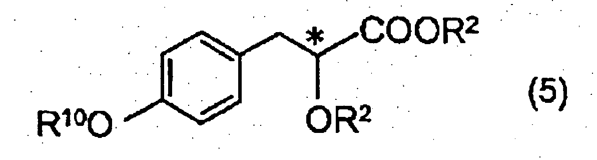 is obtained as the compound (3).

In the compounds (4) and (5), R², R¹⁰ and * are as defined above.

In this case, the proportion of the compound (4) and/or (5) depends on the reaction conditions in the alkylation of the hydroxyl group of the compound (2).

The present inventors found that the compound (4) can be obtained highly selectively as a result of selective alkylation of the hydroxyl group as attained by carrying out the alkylation reaction in the presence of a base, using an ether type solvent and/or a nitrile type solvent as the reaction solvent and an alkyl halide as the alkylating agent.

As the alkyl halide, there may be mentioned, for example, such alkyl halides as methyl iodide, ethyl iodide, methyl bromide, ethyl bromide, methyl chloride and ethyl chloride. Among them, methyl iodide, ethyl iodide, methyl bromide and ethyl bromide are preferred.

The amount of the alkyl halide to be used is not particularly restricted but, generally, the reaction can be carried out using the alkyl halide in an amount of not smaller than 1 mole equivalent relative to the compound (2). Generally speaking from the economical viewpoint, that amount is preferably not larger than 10.0 mole equivalents, more preferably not larger than 5.0 mole equivalents, still more preferably not larger than 4.0 mole equivalents.

As for the reaction solvent to be used in the above reaction; mention may be made of ether type solvents such as tetrahydrofuran and 1, 4-dioxane and nitrile type solvents such as acetonitrile and propionitrile for selective synthesis of the compound (4) Among them, tetrahydrofuran and acetonitrile are preferred and, in particular, tetrahydrofuran is preferred, from the economical viewpoint. A mixed solvent composed of these may also be used.

When, on the other hand, N,N-dimethylformamide and/or N,N-dimethylacetamide is used as the reaction solvent, the formation of the compound (4) is inhibited no matter what other conditions are and, therefore, the compound (5) can be obtained highly selectively. For selectively synthesizing the compound (5), N,N-dimethylformamide and/or N,N-dimethylacetamide is preferred. When a mixed solvent composed thereof is used, the mixing ratio is not particularly restricted.

The base species and the amount thereof, the reaction temperature, reaction concentration and reaction time, the after-treatment method and the method of deprotection in case of deprotection, which are to be used or employed in carrying out this reaction are all the same as those in the above-mentioned reaction from the compound (2) to the compound (3).

Those compounds of the formula (3) in which R¹⁰ is tert-butyl group, namely optically.active 2-substituent-oxy-3-(4-tert-butoxyphenyl)propionic acid derivatives represented by the general formula (8): 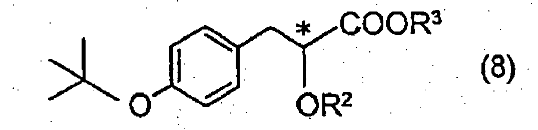 are novel compounds useful as intermediates for synthesizing medicinal compounds. In the compounds (8), R², R³ and * are as defined above. In view of the utility as intermediates for medicinal compounds, R² is preferably methyl or ethyl group, while R³ is preferably a hydrogen atom or methyl, ethyl or isopropyl group, more preferably a hydrogen atom or methyl or ethyl group. Although the absolute configuration may be either S or R, those compounds whose configuration is S are preferred.

Now, the step of producing the compound (4) by hydrolyzing the compound (5), if necessary followed by elimination of R¹⁰ (deprotection), is described.

Generally, this reaction is carried out in the presence of an acid or base. Either of the sets of reaction conditions may be employed. The acid to be used includes mineral acids such as hydrochloric acid, sulfuric acid and nitric acid, and organic acids such as acetic acid, trifluoroacetic acid, methanesulfonic acid and p-toluenesulfonic acid. Among them, hydrochloric acid and sulfuric acid are preferred from the economical viewpoint.

The base to be used includes inorganic bases such as lithium hydroxide, sodium hydroxide, potassium hydroxide, calcium hydroxide, potassium carbonate and sodium carbonate. Among them, sodium hydroxide and potassium hydroxide are preferred from the economical viewpoint.

The amount of the acid or base to be used is not particularly restricted but, generally, the reaction can be carried out using the acid or base in an amount of not smaller than 0.1 mole equivalent, relative to the compound (5). Generally speaking from the economical viewpoint, the acid or base is preferably used in an amount of not larger than 10.0 mole equivalents, more preferably not larger than 3.0 mole equivalents, still more preferably not larger than 1.5 mole equivalents. In particular, when a base is used, the reaction is carried out in most cases using the base in an amount of not smaller than 1.0 mole equivalent.

The reaction solvent for this reaction is not particularly restricted and may be any solvent incapable of adversely affecting the reaction: Thus, for example, mention may be made of hydrocarbon type solvents such as pentane, hexane, heptane, cyclohexane and petroleum ether, aromatic hydrocarbon type solvents such as toluene, benzene and xylene, nitrile type solvents such as acetonitrile and propionitrile, ether type solvents such as tert-butyl methyl ether, diethyl ether, diisopropyl ether, tetrahydrofuran and dioxane, amide type solvents such as N,N-dimethylformamide and N,N-dimethylacetamide, sulfoxide type solvents such as dimethyl sulfoxide, halogenated hydrocarbon type solvents such as methylene chloride, 1,2-dichloroethylene, chloroform and carbon tetrachloride, carboxylic acid type solvents such as acetic acid and formic acid, alcohol type solvents such as methanol, ethanol and isopropanol, and water. These solvents may also be used in the form of mixtures of two or more of them. Among them, water, methanol, ethanol and isopropanol or mixed solvents composed of two or more of them are preferred from the yield viewpoint. When a mixed solvent is used, the mixing ratio is not particularly restricted.

Although the concentration of the compound (5) in carrying out the reaction may vary depending on the reaction solvent used, the reaction can be carried out generally at a concentration of 1 to 50% by weight, preferably 5 to 30% by weight.

The reaction temperature in carrying out the reaction may vary depending on the acid or base species used and the amount thereof and on the reaction solvent species. Generally, however, it is within the range of from the freezing point to the boiling of the reaction solvent. For driving the reaction to completion in a short time, it is recommended that the reaction be carried out at an elevated temperature and, from the side reaction inhibition viewpoint, it is recommended that the reaction temperature be set at a low level. Generally, the reaction temperature is preferably within the range of -40 to 100°C, more preferably -20 to 80°C.

The reaction time for the reaction may vary depending on the acid or base species used and the amount thereof, the reaction solvent species and the reaction temperature. When the reaction is carried out at a temperature of -20 to 80°C, the reaction time is generally about 1 to 24 hours.

As for the after-treatment following this reaction, an ordinary treatment process for recovering the product from the reaction mixture can be employed. For example, water, hydrochloric acid or an aqueous alkali solution, for instance, is added to the reaction mixture after completion of the reaction for neutralization, followed by an extraction procedure using an ordinary solvent such as ethyl acetate, diethyl ether, methylene chloride, toluene or hexane. Since the product is a carboxylic acid, the extraction is generally carried out under acidic conditions. Distilling off the reaction solvent and extraction solvent from the extract obtained under reduced pressure gives the desired product. If necessary, the thus-obtained product may be subjected to an ordinary purification process such as silica gel chromatography, distillation and/or recrystallization to further increase the purity.

In this step, the protective group R¹⁰ may be eliminated (for deprotection) according to need. The deprotection can be carried out in the same manner as the above-mentioned deprotection by elimination of R¹. When the deprotection is carried out using an acid, the above-mentioned ester hydrolysis and the deprotection can also be carried out simultaneously. In cases where the compound (2) is converted to the compound (5) and the compound (5) is then converted to the compound (4) by hydrolysis, the deprotection by elimination of the protective group R¹ (or R¹⁰) can be carried out at any time after the alkylation reaction of the hydroxyl group of the compound (2). Generally, the deprotection is carried out prior to the hydrolysis of the compound (5) but may also be carried out after the hydrolysis or simultaneously with the hydrolysis.

Now, the step of etherifying the compound (4), if necessary followed by elimination (for deprotection) of R¹⁰ , to thereby produce the corresponding compound, namely optically active
2-substituent-oXy-3-(4-subsituent-oxyphenyl)propionic acid ester, represented by the general formula (6): 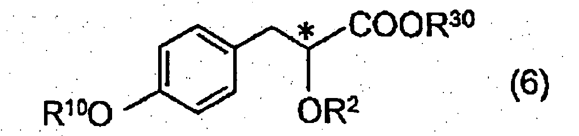 is described.

The esterification of the compound (4) can be carried out in the same manner as in the esterification reaction of the carboxyl group in the compound (2) already described hereinabove.

In this step, the protective group R¹⁰ may be eliminated (for deprotection) according to need. The deprotection can be carried out in the same manner as the elimination (for deprotection) of R¹ described hereinabove. When an acid is used in the deprotection, the deprotection can be effected simultaneously with the above-mentioned esterification reaction. In cases where the compound (2) is converted to the compound (4) and the compound (4) is then esterified to give the compound (6), the deprotection by elimination of the protective group R¹ (or R¹⁰) can be carried out at any time after the alkylation reaction of the hydroxyl group of the compound (2). Generally, the deprotection is carried out prior to the esterification of the compound (4) but may also be carried out after the esterification or simultaneously with the esterification.

Now, the step of subjecting the optically active 2-substituent-oxy-3-(4-substituent-oxyphenyl)propionic acid ester (5) to transesterification, if necessary followed by elimination (for deprotection) of R¹⁰, to produce the corresponding optically active 2-substituent-oxy-3-(4-substituent-oxyphenyl)propionic acid ester (6) is described.

The method of carrying out the transesterification of the compound (5) is not particularly restricted and the reaction can be carried out in the conventional manner. Thus, for example, there may be mentioned the method comprising adding such an acid as sulfuric acid or p-toluenesulfonic acid or such a Lewis acid as titanium tetrachloride to a mixture of an alcohol and the compound (5) and the method comprising adding a base such as sodium hydroxide or potassium carbonate to such mixture and, from the economical viewpoint, the method comprising adding an acid to the mixture of an alcohol and the compound (5) is preferred.

The reaction solvent to be used in this reaction is not particularly restricted and may be any solvent incapable of adversely affecting the reaction. Thus, mention may be made of, for example, hydrocarbon type solvents such as pentane, hexane, heptane, cyclohexane and petroleum ether, ester type solvents such as ethyl acetate and methyl acetate, aromatic hydrocarbon type solvents such as toluene, benzene and xylene, nitrile type solvents such as acetonitrile and propionitrile, ether type solvents such as tert-butyl methyl ether, diethyl ether, diisopropyl ether, tetrahydrofuran and dioxane, amide type solvents such as N,N-dimethylformamide and N,N-dimethylacetamide, sulfoxide type solvents such as dimethyl sulfoxide, halogenated hydrocarbon type solvents such as methylene chloride, 1,2-dichloroethylene, chloroform and carbon tetrachloride, carboxylic acid type solvents such as acetic acid and formic acid, alcohol type solvents such as methanol, ethanol and isopropanol, and water. Among them, alcohol type solvents such as methanol, ethanol and isopropanol are preferred. These solvents may also be used in the form of mixtures of two or more of them. When a mixed solvent is used, the mixing ratio is not particularly restricted.

Although the concentration of the compound (5) in carrying out the reaction may vary depending on the reaction solvent used, the reaction can be carried out generally at a concentration of 1 to 50% by weight, preferably 5. to 30% by weight.

The reaction temperature in carrying out the reaction may vary depending on the acid or base species used and the amount thereof and on the reaction solvent species used. Generally, however, it is within the range of from the freezing point to the boiling point of the reaction solvent. For driving the reaction to completion in a short period of time, it is recommended that the reaction be carried out at an elevated temperature and, from the side reaction inhibition viewpoint, it is recommended that the reaction temperature be set at a low level. Generally, the reaction temperature is preferably within the range of -40 to 100°C, more preferably -20 to 80°C.

The reaction time for the reaction may vary depending on the acid or base species used and the amount thereof, the reaction solvent species and the reaction temperature. When the reaction is carried out at a temperature of -20 to 80°C, the reaction time is generally about 1 to 24 hours.

This reaction step may be carried out in one stage, or the compound (5) may be hydrolyzed to give the compound (4), which is to be esterified to give the compound (6) . The method of hydrolyzing the compound (5) and the method of esterifying the compound (4) are the same as those already mentioned hereinabove.

As for the after-treatment following this reaction,an ordinary treatment process for recovering the product from the reaction mixture can be employed. For example, water, hydrochloric acid or an aqueous alkali solution, for instance, is added to the reaction mixture after completion of the reaction for neutralization, followed by an extraction procedure using an ordinary solvent such as ethyl acetate, diethyl ether, methylene chloride, toluene or hexane. Distilling off the reaction solvent and extraction solvent from the extract obtained under reduced pressure gives the desired product. If necessary, the thus-obtained product may be subjected to an ordinary purification process such as silica gel chromatography, distillation and/or recrystallization to further increase the purity.

In this step, the protective group R¹⁰ may be eliminated (for deprotection) according to need. The deprotection can be carried out in the same manner as the elimination (for deprotection) of R¹ described hereinabove. When an acid is used in the deprotection, the deprotection can be effected simultaneously with the above-mentioned transesterification reaction. In cases where the compound (2) is converted to the compound (5) and the compound (5) is then subjected to transesterification to give the compound (6), the deprotection by elimination of the protective group R¹ (or R¹⁰) can be carried out at any time after the alkylation reaction of the hydroxyl group of the compound (2). Generally, the deprotection is carried out prior to the transesterification of the compound (5) but may also be carried out after the transesterification or simultaneously with the transesterification.

When, in any step in the process of the invention, the compound is a carboxylic acid, the carboxylic acid may occur in the form of a carboxylic acid salt. As the carboxylic acid salt form, there may be mentioned alkali metal carboxylates such as sodium carboxylate, potassium carboxylate and lithium carboxylate, and alkaline earth metal carboxylates such as magnesium carboxylate and calcium carboxylate. Among them, the sodium carboxylate form is preferred.

### BEST MODE FOR CARRYING OUT THE INVENTION

The following examples illustrate the present invention in further detail. These examples are, however, by no means limitative of the scope of the invention.

### (Reference Example 1) Production of sodium 2-oxo-3-(4-tert-butoxyphenyl)propionate

A suspension of 4-tert-butoxybenzaldehyde (30.0 g, 168 mmol), hydantoin (33.73 g, 337 mmol, 2 eq.) and 1-amino-2-propanol (12.73 g, 168 mmol, 1 eq.) in distilled water (300 mL) was stirred under refluxing conditions for 4 hours. After cooling to room temperature; the precipitate was collected by filtration under reduced pressure to give 5-(4-tert-butoxybenzylidene)hydantoin (43.9 g, yield 98%).

Then, a suspension of the 5-(4-tert-butoxybenzylidene)hydantoin (30.0 g, 115 mmol) synthesized in the above manner and NaOH (24.0 g, 576 mmol, 5 eq.) in distilled water (300 mL) was stirred under refluxing conditions for 4.5 hours. After cooling to room temperature, the pH was adjusted to 7. 94 using hydrochloric acid. NaCl (35.3 g, 600 mmol) was added, and the resulting mixture was further stirring at room temperature for 15 hours. The desired product that had precipitated was collected by filtration under reduced pressure, washed with methanol and dried under vacuum to give the desired product sodium
2-oxo-3-(4-tert-butoxyphenyl)propionate (25.9 g, yield 88%).
¹H NMR (400 MHz, D₂O/ppm): δ 1.19 (s, 9H), 3.92 (s, 2H), 6.91-6.94 (m, 2H), 7.04-7.06 (m, 2H).

### (Example 1) Reduction reaction of 2-oxo-3-(4-tert-butoxyphenyl)propionic acid using lactic dehydrogenase

Sodium 2-oxo-3-(4-tert-butoxyphenyl)propionate (10 mg) synthesized in the same manner as in Reference Example, 13 mg of glucose, 1 mg of NAD⁺, 5 U of glucose dehydrogenase (product of Amano Enzyme.Inc.) and 100 U of swine heart-derived L-lactic dehydrogenase (product of Sigma) were added to a test tube containing 1.0 ml of 200 mM phosphate buffer (pH 7), and the mixture was stirred at 30°C for 16 hours. Ethyl acetate (2 mL) and 10 µl of 6 N hydrochloric acid were added and, after thorough stirring, the organic phase was recovered as an extract. The extract was analyzed under the HPLC conditions given below, and the mole conversion rate to 2-hydroxy-3-(4-tert-butoxyphenyl)propionic acid and the optical purity of the product 2-hydroxy-3-(4-tert-butoxyphenyl)propionic acid were determined. As a result, the mole conversion rate was 100%, and the absolute configuration of the 2-hydroxy-3-(4-tert-butoxyphenyl)propionic acid formed was S and the optical purity thereof was 99.8% ee.
[HPLC analysis conditions]
Column: CHIRALPAKAD-H (4.6 mm I.D. x 250 mm) (product of DAICEL CHEMICAL INDUSTRIES, LTD.), column temperature: room temperature, mobile phase; n-hexane/ethanol/trifluoroacetic acid = 950/50/1, flow rate: 1 ml/min., detection wavelength: 230 nm, retention time: R-form - 15.6 minutes, S-form - 21.3 minutes.

### (Examples 2 and 3)

The reaction procedure of Example 1 was followed in the same manner except that *Bacillus stearothermophilus-*derived L-lactic acid dehydrogenase (product of Sigma) or *Leuconostoc* mesenteroides-derived D-lactic acid dehydrogenase (product of Sigma) was used in lieu of swine heart-derived L-latic dehydrogenase (product of Sigma), followed by the same analytical procedure. As a result, when *Bacillus* stearothermophilus-derived L-lactic acid dehydrogenase was used, the mole conversion rate was 98%, the absolute configuration of the product 2-hydroxy-3-(4-tert-butoxyphenyl)propionic acid was S and the optical purity thereof was 99.8% ee. When Leuconostoc mesenteroides-derived D-lactic acid dehydrogenase was used, the mole conversion rate was 77%, the absolute configuration of the product 2-hydroxy-3-(4-tert-butoxyphenyl)propionic acid was R and the optical purity thereof was 99.5% ee.

### (Example 4) Reduction reaction of 2-oxo-3-(4-tert-butoxyphenyl)propionic acid using Bacillus stearothermophilus-derived L-lactic acid dehydrogenase

Glucose (23.5 g), 122,000 U *of Bacillus* stearothermophilus-derived L-lactic acid dehydrogenase (product of Sigma), 67, 500 U of glucose dehydrogenase (product of Amano Enzyme Inc.), 100 mg of NAD⁺ and 15.0 g of the sodium 2-oxo-3-(4-tert-butoxyphenyl)propionate synthesized in Reference Example were added to 1.5 L of 100 mM phosphate buffer (pH 6.5), and the mixture was stirred at 30°C while adjusting the pH at 6.5 by adding a 5 N aqueous sodium hydroxide solution. After 2 hours of the reaction procedure, 15.0 g of sodium 2-oxo-3-(4-tert-butoxyphenyl)propionate was further added, and the reaction was further carried out for 21 hours. After completion of the reaction, the reaction mixture was adjusted to pH 9.5 with a 6 N aqueous sodium hydroxide solution and then washed with ethyl acetate. The aqueous phase after washing was adjusted to pH 2.5 with 6 N hydrochloric acid and the product 2-hydroxy-3-(4-tert-butoxyphenyl)propionic acid was extracted with ethyl acetate. Removal of the solvent in the extract by distillation under reduced pressure gave 19.2 g of (S)-2-hydroxy-3-(4-tert-butoxyphenyl)propionic acid with an optical purity of 9.9.4% ee.

### (Example 5) Production of : (S) -2-methoxy-3-(4-tert-butoxyphenyl) propionic acid

A mixture of the (S)-2-hydroxy-3-(4-tert-butoxyphenyl)propionic acid (14.57 g, 61 mmol, optical purity 99.4% ee) synthesized in Example 4 and NaH (10.66 g, 244 mmol) in tetrahydrofuran (380 mL) was stirred under refluxing conditions for 3.5 hours. After lowering the temperature of the reaction mixture to 38°C, methyl iodide. (34.86 g, 244 mmol) was added, and the resulting mixture was further stirred for 29.5 hours. After cooling on an ice bath, 3 N hydrochloric acid (20 mL) and distilled water (25 mL) were added to terminate the reaction. The tetrahydrofuran was distilled off by concentration under reduced pressure, and the aqueous layer was washed with toluene. Toluene was added to the aqueous layer, the mixture was adjusted to pH 2.1 with 3 N hydrochloric acid (23 mL). After toluene extraction, the toluene layer was washed with distilled water and then concentrated under reduced pressure to give (S)-2-methoxy-3-(4-tert-butoxyphenyl)propionic acid (15.5 g, yield 100%, optical purity 99.4% ee) . ¹H NMR (400 MHz, CDCl₃/ppm): δ 1.33 (s, 9H), 2.94-2.99 (m, 1H), 3.08-3.13 (m, 1H), 3.39.(s, 3H), 3.95-4.00 (m, 1H), 6.90-6.96 (m, 2H), 7.12-7.14 (m, 2H).

### (Example 6) Production of

### (S)-2-metho:ky-3- (4-hydroxyphenyl) propionic acid

A mixture of the (S) -2-methoxy-3- (4-tert-butoxyphenyl) propionic acid (15.0 g, 60 mmol, optical purity 99.4% ee) synthesized in Example 5 and 3 N hydrochloric acid (290 mL) was stirred on an oil bath at 60°C for 2 hours. After ice cooling, the pH was adjusted to 2.0 with a 30% aqueous solution of NaOH, followed by extraction with ethyl acetate. Concentration of the extract under reduced pressure gave (S) -2-methoxy-3- (4-hydroxyphenyl) propionic acid (11.4 g, yield 97%, optical purity 99.4% ee).

¹H NMR (400 MHz, CDCl₃/ppm) : δ 2. 94-2. 99 (m, 1H), 3.05-3. 09 (m, 1H), 3.41 (s, 3H), 3.98-4.00 (m, 1H), 6.73-6.76 (m, 2H), 7.08-7.11 (m, 2H).

### (Example 7) Production of methyl (S)-2-methoxy-3-(4-tert-butoxyphenyl)propionate

NaH (69 mg, 1.73 mmol, 2.5 eq.) was added to a solution of (S)-2-hydroxy-3-(4-tert-butoxyphenyl)propionic acid (165.4 mg, 0.69 mmol, optical purity 99.6% ee) synthesized in the same manner as in Example 4 in dimethylformamide (3.5 mL) with ice cooling. After 10 minutes, the temperature was raised to room temperature and the mixture was then stirred at room temperature for 1 hour. Then, the mixture was stirred on a water bath at 50°C for 40 minutes and then cooled to -10°C, and methyl iodide (393.7 mg, 2.89 mmol, 4 eq.) was added dropwise. After 20 hours of stirring at -10°C, the reaction was terminated by adding a saturated aqueous solution of ammonium chloride (3.5 mL). After addition of 1 mL of distilled water, the mixture was adjusted to pH 9.52 with a 30% aqueous solution of sodium hydroxide. After extraction with toluene, the organic layer was dried over sodium sulfate. After removal of the drying agent by filtration under reduced pressure, the filtrate was concentrated under reduced pressure. The crude product obtained was purified by silica gel column chromatography (silica gel 10.23 g, eluent: hexane/ethyl acetate = 15/1), whereupon methyl

### (S)-2-methoxy-3-(4-tert-butoxyphenyl)propionate was obtained (133.9 mg, yield 73%, optical purity 99.1% ee).

¹H NMR (400 MHz, CDCl₃/ppm): δ 1.33 (s, 9H), 2.96-2.98 (m, 1H), 3.34 (s, 3H), 3. 69 (s, 3H), 3.93-3.96 (m, 1H), 6. 89-6. 91 (m, 2H), 7.09-7.11 (m, 2H) .

### [HPLC conditions for optical purity determination]

Column: OJ-RH (two columns used), column temperature: 30°C, mobile phase: phosphate buffer (pH 2) /acetonitrile = 6/4, flow rate: 0.5 ml/min., detection wavelength: 210 nm, retention time: R-form - 20.9 minutes, S-form - 21.8 minutes.

### (Example 8) Production of (S)-2-methox_y-3-(4-hydroxyphenyl)propionic acid

A mixture of methyl (S)-2-methoxy-3-(4-tert-butoxyphenyl)propionate (83 mg, 0.31 mmol) synthesized in the same manner as in Example 7 and 3 N hydrochloric acid (1 mL) was stirred at room temperature for 17 hours and then at 60°C for 4 hours . HPLC assay of the reaction mixture revealed the formation of (S)-2-methoxy-3-(4-hydroxyphenyl)propionic acid (58.8 mg, yield 87%).

### (Example 9) Production of methyl (S)-2-methoxy-3-(4-tert-butoxyphenyl)propionate and methyl (S)-2-hydroxy-3-(4-tert-butoxyphenyl)propionate

NaH. (83.2 mg, 2.08 mmol, 4 eq.) was added to a solution of (S)-2-hydroxy-3-(4-tert-butoxyphenyl)propionic acid (188.3 mg, 0.52 mmol, optical purity 99.6% ee) synthesized in the same manner as in Example 2 in dimethylformamide (2.6 mL) at room temperature, and the mixture was then stirred at room temperature for 1 hour and, after temperature raising to 46°C, further stirred for 30 minutes. After cooling to-5°C, dimethyl sulfate. (267.7 mg, 2.08 mmol, 4 eq.) was added dropwise and after 21 hours of stirring at -5°C, a saturated aqueous ammonium chloride solution (2.6 mL) was added to terminate the reaction. After addition of 5 mL of distilled water, the reaction mixture was adjusted to pH 9.65 using a 30% aqueous solution of sodium hydroxide. After extraction with toluene, the organic layer was dried over sodium sulfate. After removal of the drying agent by filtration under reduced pressure, the filtrate was concentrated under reduced pressure, and the concentrate was subjected to HPLC analysis, which indicated the formation of methyl (S)-2-methoxy-3-(4-tert-butoxyphenyl)propionate (91.4 mg, yield 66%, optical purity 98.9% ee) and methyl (S)-2-hydroxy-3-(4-tert-butoxyphenyl)propionate (7.1 mg, yield 5.4%). The formation of (S)-2-methoxy-3-(4-tert-butoxyphenyl)propionic acid was not revealed.

### [Methyl (S)-2-hydroxy-3-(4-tert-butoxyphenyl)propionate]

¹H NMR (400 MHz, CDCl₃/ppm) : δ 1.33 (s, 9H), 2.69 (d, J = 6.35 Hz, 1H), 2.90-2.96 (m, 1H), 3.05-3.10 (m, 1H), 3.75 (s, 3H), 4.41-4.46 (m, 1H), 6.90-6.92 (m, 2H), 7.09-7.11 (m, 2H).

### [HPLC analysis conditions]

Column: Hydrosphere, column temperature: 30°C, mobile phase: phosphate buffer (pH 2) /acetonitrile = 7/3, flow rate: 1 ml/min., detection wavelength: 210 nm, retention time: methyl (S)-2-methoxy-3-(4-tert-butoxyphenyl)propionate = 22.2 minutes, methyl.

### (S)-2-hydroxy-3-(4-tert-butoxyphenyl)propionate = 9.6 minutes.

### (Example 10),_ Production of methyl (S)-2-methoxy-3-(4-tert-butoxyphenyl)propionate

NaH (52 mg, 1.3 mmol, 2.5 eq.) was added to a solution of (S)-2-hydroxy-3-(4-tert-butoxyphenyl)propionic acid (124.8 mg, 0.52 mmol) synthesized in the same manner as in Example 4 in dimethylacetamide (2.6 mL) at room temperature. The mixture was then stirred at room temperature for 1 hour and, after temperature raising to 48°C, further stirred for 30 minutes. After cooling to -10°C, methyl iodide (295.2 mg, 2.08 mmol, 4 eq.) was added dropwise. After 20 hours of stirring at -10°C, a saturated aqueous ammonium chloride solution (2.6 mL) was added to terminate the reaction. After addition of 1.1 mL of distilled water, the reaction mixture was adjusted to pH 9.65 using a 30% aqueous solution of sodium hydroxide. After extraction with toluene, the organic layer was dried over sodium sulfate. The drying agent was removed by filtration under reduced pressure, the filtrate was concentrated under reduced pressure, and the concentrate was subjected to HPLC assaying, which revealed the formation of methyl (S)-2-methoxy-3-(4-tert-butoxyphenyl)propionate (103.0 mg, yield 79%). Neither methyl (S)-2-hydroxy-3-(4-tert-butoxyphenyl)propionate nor (S)-2-methoxy-3-(4-tert-butoxyphenyl)propionic acid was formed.

### (Example 11) Production of methyl

### (S)-2-methoxy-3-(4-tert-butoxyphenyl)propionate and methyl (S)-2-hydroxy-3-(4-tert-butoxyphenyl)propionate

A solution of (S)-2-hydroxy-3-(4-tert-butoXyphenyl)propionie acid (124.8 mg, 0.52 mmol) synthesized in the same manner as in Example 2 and dimethyl sulfate (267.7 mg, 2.08 mmol, 4 eq.) in tetrahydrofuran (2.6 mL) was added dropwise to a solution of NaH (52 mg, 1.3 mmol, 2.5 eq.) in tetrahydrofuran (1 mL) under refluxing conditions. The resulting mixture was then stirred under refluxing conditions for 3 hours. HPLC assaying revealed the formation of methyl (S)-2-methoxy-3-(4-tert-butoxyphenyl)propionate (90.0 mg, yield 69%), methyl (S)-2-hydroxy-3-(4-tert-butoxyphenyl)propionate (23.7 mg, yield 18%) and (S)-2-methoxy-3-(4-tert-butoxyphenyl)propionic acid (0.4 mg, yield 0.3%).

### (Example 12) Production of methyl (S) -2-methoxy-3- (4-methoxyphenyl)propionate and (S)-2-inethoxy-3-(4-methox henyl)propionic acid

NaH (100 mg, 2.5 mmol, 2.5 eq.) was added to a solution of (S) -2-hydroxy-3-(4-methoXyphenyl) propionic acid (196.2 mg, 1.0 mmol, optical purity 79.% ee) in dimethylformamide (5 mL) with ice cooling, and the mixture was then stirred at -10°C for 1.8 hours, then at room temperature for 0.75 hour and further at -10°C for 0.7 hour. At -10°C, methyl iodide (575.8 mg, 4.0 mmol, 4 eq.) was added, and the resulting mixture was further stirred at -10°C for 1.9 hours. A saturated aqueous ammonium chloride, solution (5 mL) was added to terminate the reaction. The reaction mixture was extracted with ethyl acetate, and the extract was concentrated under reduced pressure. Purification by silica gel column chromatography (eluent: hexane/ethyl acetate = 5/1) gave methyl
(S)-2-methoxy-3-(4-methoxyphenyl)propionate (91.2 mg, yield 41%, optical purity 79% ee). The aqueous layer was subjected to HPLC assaying and found to contain
(S)-2-methoxy-3-(4-methoxyphenyl)propionic acid (86.2 mg, yield 41%).

### [Methyl (S)-2-methoxy-3-(4-methoxyphenyl)propionate]

¹H NMR (400 MHz, CDCl₃/ppm): δ 2.91-3.01 (m, 2H), 3.35 (s, 3H), 3.72 (s, 3H), 3. 79 (s, 3H), 3.90-3.96 (m, 1H), 6.81-6.84 (m, 2H), 7.12-7.14 (m, 2H).

### [(S)-2-Methoxy-3-(4-methoxyphenyl)propionic acid]

¹H NMR (400 MHz, CDCl₃/ppm): δ 2.94-2.99 (m, 1H), 3.07-3.11 (m, 1H), 3.40 (s, 3H), 3.79 (s, 3H), 3.97-4.00 (m, 1H), 6.82-6.85 (m, 2H), 7.15-7.17 (m, 2H)

### [HPLC assay conditions]

Column: Lichrosphere, column temperature: 30°C, mobile phase: phosphate buffer (pH 2) /acetonitrile = 8/2) , flow rate: 1 ml/min., detection wavelength: 210 nm, retention time: 23.6 minutes.

### [HPLC conditions for optical purity determination]

Column: OD-H (two columns used), column temperature: 30°C, mobile phase: n-hexane/2-propanol = 95/5, flow rate: 0.5 ml/min., detection wavelength: 210 nm, retention time: (R) -form - 20.9 minutes, (S)-form - 21.8 minutes.

### (Example 13) Production of (S)-2-methoxy-3-(4-methoxyphenyl)propionic acid

A solution of NaOH (1.06 g, 27.8 mmol, 1.2 eq.) in water (39.5 mL) as well as methanol (38 mL) was added to methyl (S)-2-methoxy-3-(4-methoxyphenyl) propionate (5.16 g, 2 3 mmol, optical purity 87% ee) synthesized in the same manner as in Example 12, with ice cooling. After 2.7 hours of stirring at room temperature, the reaction mixture was washed with toluene. The aqueous layer was adjusted to pH 2.02 by addition of hydrochloric acid and then extracted with ethyl acetate. Concentration under reduced pressure gave (S)-2-methoxy-3-(4-methoxyphenyl)propionic acid (4.63 g, yield 96%, optical purity 87% ee). The optical purity of the (S)-2-methoxy-3-(4-methoxyphenyl)propionic acid was determined after derivatization thereof to methyl (S)-2-methoxy-3-(4-methoxyphenyl)propionate using trimethylsilyldiazomethane.
¹H NMR (400MHz, CDCl₃/ppm): δ 2.94-2.99 (m, 1H), 3.06-3.11 (m, 3H), 3.40 (s, 3H), 3.79 (s, 3H), 3.97-4.00 (m, 1H), 6.82-6.85 (m, 2H), 7.13-7.17 (m, 2H).

### (Example 14) Production of (S)-2-methoxy-3-(4-hydroxyphenyl)propionic acid

A solution of the (S)-2-methoxy-3-(4-methoxyphenyl)propionic acid (525.5 mg, 2.5 mmol, optical purity 87% ee) synthesized in Example 13, KOH (632.1 mg, 11.3 mmol, 4.5 eq.) and octanethiol (1.28 g, 8.75 mmol, 3.5 eq.) in 1-methyl-2-pyrrolidone (2.6 mL) was stirred at 120°C for 46 hours. After addition of distilled water (2 mL), the mixture was cooled to room temperature and adjusted to pH 6. 9 with 4 N hydrochloric acid (1.8 mL) . After extraction with ethyl acetate, the ethyl acetate layer was washed with water. Concentration under reduced pressure gave (S)-2-methoxy-3-(4-hydroxyphenyl)propionic acid (451.3 mg, yield 92%, optical purity 87% ee).

### (Example 15) Production of (S)-2-ethoxy-3-(4-tert-butoxyphenyl)propionic acid and ethyl (S)-2-ethoxy-3-(4-tert-butoxyphenyl)propionate

A mixture of (S)-2-hydroxy-3-(4-tert-butoxyphenyl)propionic acid (119.2 mg, 0.5 mmol) synthesized in the same manner as in Example 2, 55% NaH (87.9 mg, 2 mmol) and dimethylformamide (2.5 mL) was stirred at room temperature for 1 hour and then further at 50°C for 1 hour. After lowering the temperature of the reaction mixture to -15°C, ethyl iodide (329.8 mg, 2 mmol) was added, and the resulting mixture was further stirred for 23 hours. A saturated aqueous ammonium chloride solution and water were added thereto (pH 9.0) and the whole mixture was extracted with ethyl acetate. The extract was dried over magnesium sulfate, the solvent was distilled off under reduced pressure and the residue was purified by silica gel column chromatography to give ethyl (S)-2-ethoxy-3-(4-tert-butoxyphenyl)propionate (53.2 mg, yield 36%). The aqueous layer from the above extraction was adjusted to pH 1.9 by addition of 3 N hydrochloric acid, followed by extraction with ethyl acetate. The extract was dried over magnesium sulfate and the solvent was distilled off under reduced pressure to give (S)-2-ethoxy-3-(4-tert-butoxyphenyl)propionic acid (63.9 mg, yield 48%).

### [(S)-2-Ethoxy-3-(4-tert-butoxyphenyl)propionic acid]

¹H NMR (400 MHz, CDCl₃/ppm) : δ 1.18 (t, 3H), 1.34 (s, 9H), 2.91-2.97 (m, 2H), 3.36-3.63 (m, 2H), 4.04 (m, 1H), 6.91-6.93 (m, 2H), 7.13-7.15 (m, 2H).

### [Ethyl (S)-2-ethoxy-3-(4-tert-butoxyphenyl)propionate]

¹H NMR.(400 MHz, CDCl₃/ppm): δ 1.13-1.20 (m, 6H), 1.34 (s, 9H), 2.92-3.00 (d, 2H), 3.31-3.64 (m, 2H), 3.95-4.00 (m, 1H), 4.12-4.21 (q, 2H), 6:89-6.92 (m, 2H), 7.12-7.14 (m, 2H).

### (Example 16) Production of (S)-2-ethoxy-3-(4-tert-butoxyphenyl)propionic acid

A mixture of (S)-2-hydroxy-3-(4-tert-butoxyphenyl)propionic acid (238.8 mg, 1 mmol) synthesized in the same manner as in Example 2, 55% NaH (174.5 mg, 4 mmol) and tetrahydrofuran (6.3mL) was stirred under refluxing conditions for 4 hours. After lowering the temperature of the reaction mixture to 40°C, ethyl iodide (657 .1 mg, 4 mmol) was added and the whole mixture was further stirred for 22 hours. After ice cooling, 3 N hydrochloric acid and distilled water were added to terminate the reaction and make the pH 1.7. The resulting mixture was extracted with ethyl acetate, the extract was dried over magnesium sulfate, and the solvent was distilled off under reduced pressure to give (S)-2-ethoxy-3-(4-tert-butoxyphenyl)propionic acid (55.9 mg, yield 21%). The formation of ethyl (S)-2-ethoxy-3-(4-tert-butoxyphenyl)propionate was not observed in this reaction.

### (Example 17) Production of (S)-2-ethoxy-3-(4-tert-butoxyphenyl)propionic acid

A mixture of (S)-2-hydroxy-3-(4-tert-butoxyphenyl)propionic acid (119.1 mg, 0.5 mmol) synthesized in the same manner as in Example 2, 55% NaH (87.9 mg, 2 mmol) and tetrahydrofuran (3.2 mL) was stirred under refluxing conditions for 4 hours. After lowering the temperature of the reaction mixture to 40°C, ethyl bromide (155 µl, 2 mmol) was added and the whole mixture was further stirred for 22 hours. After ice cooling, a saturated aqueous ammonium chloride solution and water were added (pH9.0), and the resulting mixture was washed with ethyl acetate. The aqueous layer was adjusted to pH 2.0 with 3 N hydrochloric acid and extracted with ethyl acetate, the extract was dried over magnesium sulfate, and the solvent was distilled off under reduced pressure to give
(S)-2-ethoxy-3-(4-tert-butoxyphenyl)propionic acid (17.3 mg, yield 13%). The formation of ethyl
(S)-2-ethoxy-3-(4-tert-butoxyphenyl)propionate was not observed in this reaction.

## Claims

1. A process for producing an optically active 2-.substituent-oxy-3-(4-substituent-oxyphenyl)propionic acid derivative represented by the general formula (3): 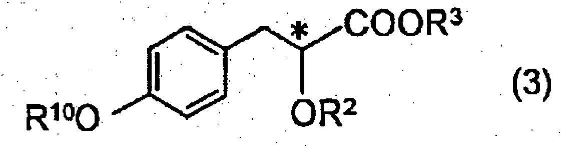 (wherein * indicates an asymmetric carbon atom, R¹⁰ represents a hydrogen atom or an ether type protective group represented by R¹, R² represents an alkyl group containing 1 to 10 carbon atoms, which may optionally be substituted, or an aralkyl group containing 7 to 20 carbon atoms, which may optionally be substituted, R³ represents a hydrogen atom, an alkyl group containing 1 to 10 carbon atoms, which may optionally be substituted, an aryl group containing 6 to 20 carbon atoms, which may optionally be substituted, or an aralkyl group containing 7 to 20 carbon atoms, which may optionally be substituted)
which comprises stereoselectively reducing an 2-oxo-3-(4-substituent-oxyphenyl)propionic acid represented by the general formula (1): 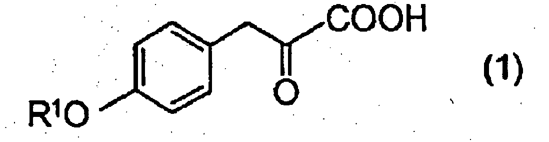 (wherein R¹ represents an ether type protective group) by means of an enzyme, and subjecting the thus-obtained optically active 2-hydroxy-3-(4-substituent-oxyphenyl)propionic acid represented by the general formula (2): 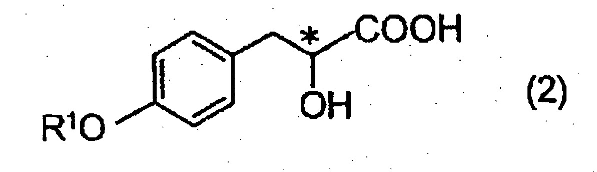 (wherein * and R¹ are as defined above) to esterification of the carboxyl group according to need, then to alkylation of the hydroxyl group and, if necessary, to elimination of R¹ (deprotection).

2. The process according to Claim 1
which comprises alkylating the hydroxyl group of an optically active
2-hydroxy-3-(4-substituent-oxyphenyl)propionic acid represented by the general formula (2) given above in an ether type solvent and/or a nitrile type solvent in the presence of a base in order to produce an optically active
2-substituent-oxy-3-(4-substituent-oxyphenyl)propionic acid represented by the general formula (4): 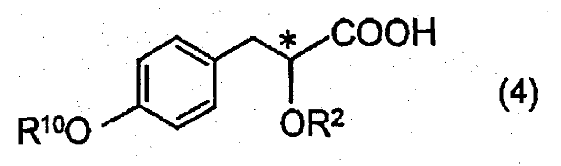 (wherein *, R¹⁰ and R² are as defined above): as the compound represented by the general formula (3) given above.

3. The process according to Claim 1
which comprises alkylating the hydroxyl group or the carboxyl group of an optically active
2-hydroxy-3- (4-substituent-oxyphenyl)propionic acid represented by the general formula (2) given above in order to produce an optically active
2-substituent-oxy-3-(4-subsituent-oxyphenyl)propionic acid ester represented by the general formula (5): 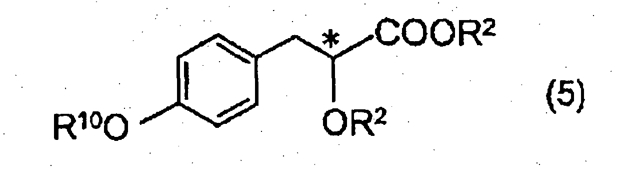 (wherein *, R¹⁰ and R² are as defined above): as the compound represented by the general formula .(3) given above.

4. The process according to Claim 1
which comprises esterifying the carboxyl group of an optically active
2-hydroxy-3-(4-substituent-oxyphenyl)propionic acid represented by the general formula (2) given above and then alkylating the hydroxyl group in order to produce an optically active
2-substituent-oxy-3-(4-subsituent-oxyphenyl)propionic acid ester represented by the general formula (6): 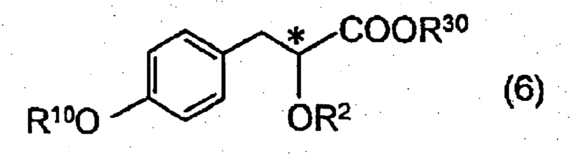 (wherein *, R¹⁰ and R² are as defined above, R³⁰ represents an alkyl group containing 1 to 10 carbon atoms, which may optionally be substituted, an aryl group containing 6 to 20 carbon atoms, which may optionally be substituted, or an aralkyl group containing 7 to 20 carbon atoms, which may optionally be substituted): as the compound represented by the general formula (3-) given above.

5. A process for producing an optically active 2-substituent-oxy-3- (4-substituent-oxyphenyl) propionic acid represented by the general formula (4): 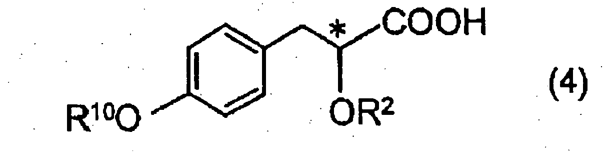 (wherein *, R¹⁰ and R² are as defined above)
which comprises hydrolyzing the ester group of the compound represented by the general formula (5), which is obtained in the process according to Claim 3, if necessary followed by elimination of R¹⁰ (deprotection).

6. A process for producing an optically active 2-substituent-oxy-3-(4-subsituent-oxyphenyl)propionic acid ester represented by the general formula (6): 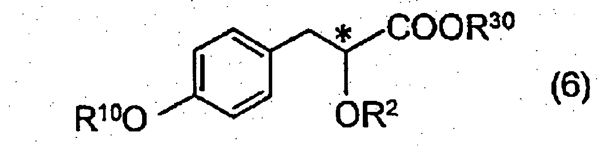 (wherein *, R¹⁰, R² and R³⁰ are as defined above)
which comprises esterifying the compound represented by the general formula (4), which is obtained in the process according to Claim 2, if necessary followed by elimination of R¹⁰(deprotection) .

7. A process for producing an optically active 2-substituent-oxy-3-(4-subsituent-oxyphenyl)propionic acid ester represented by the general formula (6): 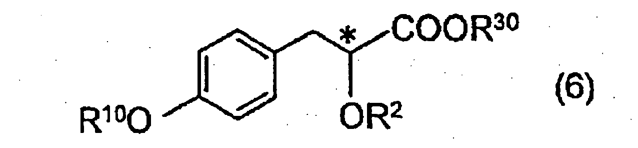 (wherein *, R¹⁰, R² and R³⁰ are as defined above)
which comprises subjecting the compound represented by the general formula (5) given above, which is obtained in the process according to Claim 3, to transesterification, if necessary followed by elimination of R¹⁰ (deprotection).

8. The process according to any one of Claims 1 to 7
wherein the enzyme is an α-keto acid reducing enzyme.

9. The process according to any one of Claims 1 to 8
wherein the enzyme is an lactic acid dehydrogenase, hydroxyisocaproic acid dehydrogenase, or an mandelic acid dehydrogenase.

10. The process according to any one of Claims 1 to 7
wherein the enzyme is L- or D-lactic acid dehydrogenase.

11. A process for producing an optically active 2-substituent-oxy-3-(4-substituent-oxyphenyl)propionic acid represented by the general formula (4): 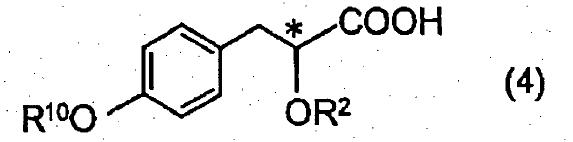 (wherein * indicates an asymmetric carbon atom, R¹⁰ represents a hydrogen atom or the same as R¹, namely an ether type protective group, R² represents an alkyl group containing 1 to 10 carbon atoms, which may optionally be substituted, or an aralkyl group containing 7 to 20 carbon atoms, which may optionally be substituted)
which comprises selectively alkylating the hydroxyl group of an optically active
2-hydroxy-3-(4-substituent-oxyphenyl)propionic acid represented by the general formula (2): 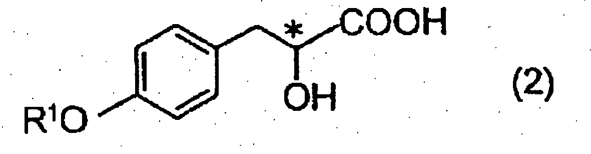 (wherein * is as defined above, R¹ represents an ether type protective group) : with an alkyl halide in an ether type solvent and/or a nitrile type solvent in the presence of a base, if necessary followed by elimination of R¹ (deprotection).

12. The process according to any one of Claims 1 to 11
wherein R¹ is methyl group, tert-butyl group, benzyl group, allyl group, methoxymethyl group, methoxyethoxymethyl group, tetrahydropyranyl group, benzyloxymethyl group, methylthiomethyl group, 2-(trimethylsilyl)ethoxymethyl group, or tert-butoxymethyl group.

13. The process according to any one of Claims 1 to 12
wherein R¹ is tert-butyl group.

14. The process according to any one of Claims 1 to 13
wherein R² is methyl group or ethyl group.

15. The process according to any one of Claims 1 to 14
wherein R¹⁰ is a hydrogen atom.

16. The process according to any one of Claims 1 to 15
wherein the absolute configuration of an optically active 2-hydroxy-3-(4-substituent-oxyphenyl)propionic acid represented by the general formula (2) given above is S.

17. The process according to any one of Claims 2, 6, and 11 to 15
wherein the ether type solvent and/or a nitrile type solvent is tetrahydrofuran and/or acetonitrile.

18. An 2-oxo-3-(4-tert-butoxyphenyl)propionic acid represented by the general formula (7): 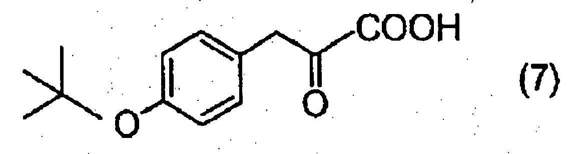 :or the salt thereof.

19. An optically active 2-substituent-oxy-3-(4-tert-butoxyphenyl)propionic acid derivative represented by the general formula (8) 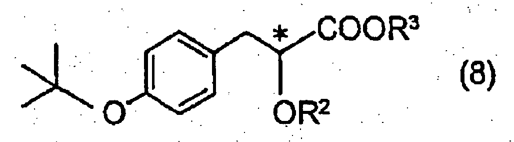 (wherein * indicates an asymmetric carbon atom, R² represents an alkyl group containing 1 to 10 carbon atoms, which may optionally be substituted, or an aralkyl group containing 7 to 20 carbon atoms, which may optionally be substituted, R³ represents a hydrogen atom, an alkyl group containing 1 to 10 carbon atoms, which may optionally be substituted, an aryl group containing 6 to 20 carbon atoms, which may optionally be substituted, or an aralkyl group containing 7 to 20 carbon atoms, which may optionally be substituted).

20. The compound according to Claim 19
wherein R² is methyl group or ethyl group.

21. The compound according to Claim 19 or 20
wherein R³ is a hydrogen atom, methyl group, ethyl group or isopropyl group.

22. The compound according to any one of Claims 19 to 21 the absolute configuration of which is S.
